# EUROPÄISCHE PATENTSCHRIFT

(11) **EP 4 593 756 B1**
(45) Veröffentlichungstag und Bekanntmachung des Hinweises auf die Patenterteilung: **10.06.2026**
(21) Anmeldenummer: 23776887.4
(22) Anmeldetag: 21.09.2023
(51) Int. Cl.: A61B 90/00, F16B 2/22, F16B 21/07, F16B 21/18

(54) **CHIRURGISCHER ROBOTER MIT ABDECKUNG**
SURGICAL ROBOT WITH COVER
ROBOT CHIRURGICAL À ÉLÉMENT DE COUVERTURE

(30) Priorität: 29.09.2022 DE 102022125216
(43) Veröffentlichungstag der Anmeldung: 06.08.2025
(73) Patentinhaber: B. Braun New Ventures GmbH, 79110 Freiburg im Breisgau (DE)
(72) Erfinder: HAHN, Lukas, 72488 Sigmaringen (DE)
(74) Vertreter: Winter, Brandl - Partnerschaft mbB
(86) Internationale Anmeldenummer: PCT/EP2023/076029
(87) Internationale Veröffentlichungsnummer: WO 2024/068416

(56) Entgegenhaltungen:
- EP-B1- 2 916 762
- EP-B1- 2 983 606
- CN-A- 113 133 830
- CN-A- 114 404 040
- US-A1- 2022 125 522
- US-B2- 11 096 754
- ANONYMOUS: "25 Stück AWAPRO Kunststoff Ösen Blau + 1 Rundlocheisen 13mm Set - Ideal für Bootsplanen, Poolfolie, LKW Planen etc. : Amazon.de: Baumarkt", 17 March 2022 (2022-03-17), XP093113848, Retrieved from the Internet <URL:https://www.amazon.de/dp/B09VV7H6Q7/ref=sspa_dk_detail_1?pf_rd_p=ae2317a0-2175-4285-af64-66539858231f&pf_rd_r=VYZXYJZT0X959P06GXKH&pd_rd_wg=XBOvJ&pd_rd_w=YInAp&content-id=amzn1.sym.ae2317a0-2175-4285-af64-66539858231f&pd_rd_r=70c2e6f2-267c-4091-8f3a-214c1cac7395&s=industrial&sp_csd=d2lkZ2V0TmFtZT1zc> [retrieved on 20231219]

## Beschreibung

### Technisches Gebiet

Die vorliegende Offenbarung betrifft einen chirurgischen Roboter für einen chirurgischen Eingriff an einem Patienten, mit einem Roboterarm und einem daran angebundenen Roboterkopf, einer Vielzahl an Navigationsmarkern, einer sterilen Abdeckfolie und einer Vielzahl von Befestigungselementen.

### Hintergrund der vorliegenden Offenbarung

Für eine Nachverfolgung/ ein Tracking eines Roboters weist dieser eine Vielzahl an Navigationsmarkern auf, etwa eine Vielzahl an (aktiven oder passiven) Leuchtdioden (LEDs). Ein Problem hierbei ist jedoch, dass der Roboter nicht steril ist und für einen chirurgischen Eingriff eine Sterilbarriere in Form eines für Strahlen, insbesondere Infrarotstrahlen, transparenten Abdecktuchs/ Abdeckfolie eingefügt werden muss. In einer besonders einfachen Variante kann ein steriles transparentes Abdecktuch über den Roboter geworfen werden, so dass dieses den Roboter gänzlich ummantelt und so eine Sterilbarriere gegenüber seiner Umwelt bereitstellt.

Genauigkeitsstudien haben jedoch gezeigt, dass die Nachverfolgungsgenauigkeit von mit Abdeckungen versehenen Infrarot-LEDs aufgrund von Lichtreflexionen an der Abdeckfolie deutlich abnimmt. Eine genaue Ausrichtung des transparenten sterilen Abdecktuchs ist daher von besonderer Bedeutung. Auch stellen eine sichere und einfache Befestigung des sterilen Abdecktuchs aktuell Probleme dar.

Nach aktuellem Stand der Technik gibt es beispielsweise einen navigierten Roboterarm mit integriertem Laser zum Schneiden von Knochenstrukturen. Dieser Knochenschneideroboter verfügt ebenfalls über drapierte LEDs, wobei das sterile Abdecktuch/ die sterile Drapierfolie/ die sterile Abdeckfolie mit einer zusammenziehbaren, umfänglichen Schnur bzw. Kordel an der Oberfläche des Roboters befestigt wird. Eine präoperative oder intraoperative Befestigung der Kordel (also vor oder während eines chirurgischen Eingriffs/ einer Operation) kann jedoch als schlecht bewertet werden, da das Überziehen des Abdecktuchs und Zusammenschnüren der Kordel komplex ist und zudem kein standardisiertes Vorgehen bereitgestellt wird. Zudem können Lichtreflexionen der Abdeckfolie im direkten Bereich um die LED zu weiteren Tracking-Ungenauigkeiten führen, da es keinen kontrastreicheren Hintergrund gibt.

Auch wird nach einem Stand der Technik eine Nachführung eines Mikroskopkopfes realisiert, jedoch geschieht dies mit Hilfe einer passiver Nachführtechnik durch Anbringen von schwarzen Schnittstellen über der Abdeckfolie (also im sterilen Bereich), an denen die passiven Marker angebracht werden können. Diese Konfiguration hat den entscheidenden Nachteil, dass bei der Aufnahme des Patienten in einem nicht sterilen Bereich auch der Mikroskopkopf verfolgt werden muss. Daher werden sowohl sterile als auch unsterile passive Marker benötigt, was erhöhte Produktions- und Sterilisierungskosten und folglich auch Operationskosten nach sich zieht und zudem eine weitere Fehlerquelle hinzufügt, etwa durch versehentliches Verwechseln von sterilen und unsterilen Markern.

Ein weiterer Stand der Technik beschreibt einen sterilen Endeffektor mit integrierten LEDs an einem unsterilen Roboterarm zu befestigen. Diese Gestaltung hat den Nachteil, dass sterilisierbare LEDs, mit entsprechend einhergehenden sehr hohe Kosten, in den Endeffektor integriert werden müssen und ferner sogar noch eine Stromversorgung für die LEDs durch die Abdeckfolie erfolgen muss. Auch beschreibt ein weiterer Stand der Technik die Integration von Fixierungselementen an mindestens einem der mehreren Glieder des Roboterarms zur Abdeckung von LEDs.

Zudem ist aus der CN 113 133 830 A ein Clip für einen optischen Tracker bekannt. Ferner ist aus der US 2022/125522 A1 eine retroreflektierende Scheibe bekannt.

Weiter offenbart die US 11,096,754 B2 ein chirurgisches Robotersystem, bei dem der Roboterarm und die davon abstehenden LEDs zur Navigation durch eine sterile Abdeckung, die mittels eines Befestigungselements lösbar anbringbar ist, abgedeckt wird. Zudem offenbart die EP 2 916 762 B1 eine zweiteilig ausgebildete Trackingstruktur, bei der ein erster Teil mit Trackingmarkern lösbar an einem zweiten Teil befestigt werden kann, wobei zwischen den beiden Teilen ein Abdecktuch eingeklemmt werden kann. Ferner offenbart die CN 114 404 040 A eine optische Trackinganordnung. Darüber hinaus offenbart die EP 2 983 606 B1 ein Kameraabdeckungssystem, bei dem eine Kamera zur Nachverfolgung von Markerelementen mit einer sterilen Abdeckung abgedeckt wird.

### Zusammenfassung der vorliegenden Offenbarung

Es ist daher die Aufgabe der vorliegenden Offenbarung, die Nachteile des Stands zu vermeiden oder zumindest zu mindern und einen chirurgischen Roboter sowie ein System aus einer sterilen Abdeckfolie und einem Abdeckungsbefestigungselement bereitzustellen, welches/ welche eine besonders einfache und sichere Befestigung sowie eine präzise Nachverfolgung von Navigationsmarkern bereitstellt.

Die Aufgaben der vorliegenden Offenbarung werden hinsichtlich des chirurgischen Roboter durch die Merkmale des Anspruchs 1 gelöst. Vorteilhafte Weiterbildungen sind Gegenstand der abhängigen Ansprüche.

Demnach betrifft die vorliegende Offenbarung einen chirurgischen Roboter für einen chirurgischen Eingriff an einem Patienten, mit einem an einer Roboterbasis angebundenen Roboterarm und einem an den Roboterarm angebundenen Roboterkopf, wobei der Roboterarm zumindest ein motorisiertes Roboterarmsegment aufweist, einer Vielzahl an Navigationsmarkern, die an einer Oberfläche des zumindest einen Roboterarmsegments und/oder an dem Roboterkopf angeordnet sind, wobei diese Navigationsmarker gegenüber der Oberfläche jeweils zumindest teilweise hervorstehen, wobei zumindest eine Teilmenge der Navigationsmarker eine LED-Befestigungsschnittstelle umfasst, eine chirurgische sterile Abdeckfolie, die so angepasst ist, dass sie über die Navigationsmarker angeordnet werden kann, und eine Vielzahl von Abdeckungsbefestigungselementen, wobei die Abdeckungsbefestigungselemente mit der chirurgischen sterilen Abdeckfolie zusammenwirken und lösbar über die Navigationsmarker über deren LED-Befestigungsschnittstelle an- und abkoppelbar gestaltet sind, wobei jedes der Abdeckungsbefestigungselemente eine Öffnung entlang einer Längsachse von einer Unterseite zu einer Oberseite des Abdeckungsbefestigungselements aufweist, in welche der Navigationsmarker einführbar ist, wobei jedes der Abdeckungsbefestigungselemente eine komplementär zu der LED-Befestigungsschnittstelle ausgebildete Abdeckungsbefestigungsschnittstelle im Bereich der Unterseite des Abdeckbefestigungselements aufweist, über welche das Abdeckungsbefestigungselement lösbar kraftschlüssig und/oder formschlüssig befestigbar ist und welche mit der LED-Befestigungsschnittstelle zusammenwirkt, um einen Teil der chirurgischen sterilen Abdeckfolie an den Navigationsmarkern zu befestigen und zu sichern.

Ein Grundgedanke der vorliegenden Offenbarung sieht also vor, für eine Befestigung einer sterilen Abdeckfolie die Navigationsmarker, insbesondere die Leuchtdioden bzw. die LED-Gehäuse der Leuchtdioden des Roboters, zu nutzen, um an diesen gewissermaßen lösbar an- und auch wieder abzudocken. Hierfür werden speziell gestaltete Abdeckungsbefestigungselemente verwendet, welche es ermöglichen, eine präzise Verfolgung von Navigationsmarkern, insbesondere von Leuchtdioden/ LEDs (light-emitting diodes), insbesondere Infrarot-LEDs, durchführen zu können. Während also die Navigationsmarker wie Leuchtdioden (LEDs) selbst nicht steril sein müssen, wird über das ankoppelbare Abdeckungsbefestigungselement eine sterile Abdeckfolie befestigt und dabei straff über den Navigationsmarker, insbesondere die Leuchtdiode, gespannt, wodurch eine glatte Oberfläche der Abdeckfolie entsteht und verhindert wird, dass die sterile Folie die Verfolgungsgenauigkeit beeinflusst (Lichtreflexionen und Beugung). Durch die Abdeckungsbefestigungselemente (insbesondere im Zusammenwirken mit der LED oder einem Gehäuse der LED) kann also eine glatte Oberfläche erzeugt und die Verfolgungsgenauigkeit des mit sterilem Tuch bzw. Folie bedeckten Navigationsmarkers, insbesondere der LEDs, deutlich verbessert werden. Die Abdeckfolie-Abdeckbefestigungselemente spannen dabei die sterile Abdeckfolie straff über die Navigationsmarker, insbesondere die Tracking-LEDs, ohne jedoch die sterile Barriere zu zerstören. Hierdurch wird eine einfache, kostengünstige, schnell befestigbare und dennoch sichere und standardisierte Schaffung einer Sterilbarriere über die entsprechende Befestigung der sterilen Abdeckfolie mittels der speziellen Abdeckungsbefestigungselemente an den Navigationsmarkern, insbesondere LEDs, des chirurgischen Roboters geschaffen. Die Navigationsmarker (wie etwa LEDs) selbst (bzw. in ihrem Gehäuse) sind fest in einen unsterilen Roboterkopf (als oder mit Endeffektor) wie einen Mikroskopkopf oder unsterilen Endeffektor integriert, der entsprechend abgedeckt wird.

Mit noch anderen Worten hat das technische Design als Grundlage eine sterile Abdeckfolie, etwa LED-Abdeckfolie, die über das spezielle Abdeckungsbefestigungselement über eine Schnappverbindung oder magnetisch an einer standardisierten Schnittstelle, insbesondere eines navigierten unsterilen Endeffektors (am oder als Roboterkopf), besonders bevorzugt eines Mikroskopkopfs, befestigt werden kann. Diese standardisierte (LED-Befestigungs-)Schnittstelle kann auch das Gehäuse des Navigationsmarkers, insbesondere das Gehäuse der (Tracking)LED, sein. Darüber hinaus verhindert der Schnappverschluss oder Magnetverschluss ein unbeabsichtigtes Lösen der Abdeckungsbefestigungselemente (etwa LED-Abdeckungen).

Mit noch ganz anderen Worten wird gemäß der vorliegenden Offenbarung ein medizinisches Abdeckungsbefestigungselement für eine lösbare Fixierung einer sterilen Abdeckfolie/ eines sterilen Abdecktuchs an einem Navigationsmarker, insbesondere einer Leuchtdiode (LED), insbesondere an einer Leuchtdiode eines chirurgischen Roboters, bereitgestellt mit: einer (Durchgangs-)Öffnung entlang einer Längsachse (des Abdeckungsbefestigungselements) von einer Unterseite zu einer Oberseite des Abdeckungsbefestigungselements, in welche von der Unterseite (des Abdeckbefestigungselements) her ein vorstehender, insbesondere rotationssymmetrischer, Körper, bevorzugt ein Navigationsmarker, ganz bevorzugt eine Leuchtdiode mit Gehäuse, in Richtung der Oberseite einführbar ist, mit einer Abdeckungsbefestigungsschnittstelle, insbesondere im Bereich der Unterseite des Abdeckbefestigungselements, über welche das Abdeckungsbefestigungselement lösbar kraftschlüssig und/oder formschlüssig (gegenüber dem vorstehenden rotationssymmetrischer Körper, insbesondere dem Navigationsmarker wie beispielsweise der LED) befestigbar (an- und abkoppelbar ausgebildet) ist, wobei das Abdeckungsbefestigungselement insbesondere steril und/oder sterilisierbar ausgebildet ist.

Der Begriff Unterseite definiert dabei die Seite des Abdeckbefestigungselements, welches der zu befestigenden Oberfläche zugewandt ist, beispielsweise der Oberfläche des chirurgischen Roboters oder des Navigationsmarkers. Dem gegenüber bildet die Oberseite des Abdeckbefestigungselements die endständige freie Seite des Abdeckbefestigungselements, welche nach außen zur Umgebung hin ausgerichtet ist.

Der Navigationsmarker dient der Nachverfolgung durch ein Navigationssystem bzw. ein Trackingsystem (beispielsweise ein optisches Trackingsystem), um zumindest eine Position des Navigationsmarkers im Raum zu bestimmen. In einer Ausführungsform kann der Navigationsmarker eine Leuchtdiode sein, welche nachverfolgbar ist.

Gemäß einer bevorzugten Ausführungsform kann das Abdeckbefestigungselement als Befestigungsclip mit Schnappverbindung als Abdeckungsbefestigungsschnittstelle ausgebildet sein, bei dem sich zumindest ein, insbesondere mehrere, Rastfinger/ Cliparm in Richtung der Längsachse zu der Unterseite hin erstreckt und in radialer Richtung elastisch auslenkbar ist, um das Abdeckungsbefestigungselement kraftschlüssig und/oder formschlüssig zu halten, wobei vorzugsweise der Rastfinger für eine Ausbildung eines Hinterschnitts für einen Formschluss einen endständigen Vorsprung nach radial innen und/oder nach radial außen aufweist. Dieser radialelastische Rastfinger bewirkt also bei einer Auslenkung nach radial außen eine elastische Vorspannung nach radial innen wie auch umgekehrt. So kann über einen Reibschluss (also Kraftschluss, wenn sich der Rastfinger elastisch ausgelenkt radial anlegt) und/oder über einen Formschluss über einen Rastvorsprung und einen Rasthinterschnitt eine Befestigung erzielt werden.

Gemäß einer weiteren Ausführungsform kann das Abdeckbefestigungselement um seine Öffnung herum gleichverteilte, insbesondere rotationssymmetrisch angeordnete, zu seiner Unterseite hin vorstehende Rastfinger aufweisen, welche eine homogene umfängliche Fixierung des Abdeckungsbefestigungselements gewährleisten und die Rastfinger insbesondere selbst die Unterseite ausbilden, also die letzten Elemente zur Unterseite hin bilden. Die umfänglich gleichverteilen Rastfinger können so radial ausgelenkt werden und beispielsweise über einen jeweiligen endständigen Rastvorsprung umfänglich einschnappen, um das Abdeckbefestigungselement zu befestigen.

Insbesondere kann das Abdeckungsbefestigungselement als Abdeckungsbefestigungsschnittstelle im Bereich der Unterseite zumindest einen Magneten für eine magnetische Verbindung aufweisen, insbesondere einen um seine Längsachse herum konzentrisch angeordneten ringförmigen Magneten aufweisen, der vorzugsweise selbst zumindest einen Teil der Unterseite des Abdeckungsbefestigungselement bildet, um das Abdeckungsbefestigungselement an einem komplementären Magneten oder an einer metallischen Oberfläche kraftschlüssig zu befestigen. In dem Abdeckbefestigungselement selbst ist also ein Magnet fest integriert, um das Abdeckbefestigungselement zumindest kraftschlüssig (magnetisch) zu befestigen.

Gemäß einer Ausführungsform eines (getrennten) magnetischen Abdeckungsbefestigungselements/ LED-Abdeckung kann also in dieser Variante ein Magnet dauerhaft in den unsterilen Endeffektor, insbesondere Mikroskopkopf, integriert sein. Das Abdeckungsbefestigungselement, etwa als Kunststoffteil, mit integriertem Magneten kann über die LED gezogen werden und die Magnetkraft schafft eine lösbare kraftschlüssige Verbindung. Dadurch wird die Abdeckfolie gespannt und eine glatte Oberfläche für eine optimale Nachführung der LED geschaffen.

Insbesondere kann das Abdeckungsbefestigungselement aus einem thermoplastischem Kunststoff mit ringförmiger Ausnehmung/Nut hergestellt sein, in welche der Magnet einsetzbar ist.

Insbesondere schließt Magnet bündig ab und wird lediglich radial außen umfasst. In dieser Ausführungsform liegt der Magnet also als Unterseite des Abdeckungsbefestigungselements frei.

Vorzugsweise kann das Abdeckungsbefestigungselement im Bereich seiner Oberseite zumindest einen, insbesondere umfänglichen, Vorsprung nach radial außen aufweisen, um einen Hinterschnitt für eine manuelle Handhabung auszubilden. Auf diese Weise kann das Abdeckbefestigungselement bei Bedarf auch gut manuell wieder entfernt werden.

Insbesondere kann das Abdeckungsbefestigungselement rotationssymmetrisch gestaltet sein und in Richtung der Längsachse insbesondere eine konische Form/ Formgebung aufweisen, wobei insbesondere ein Außendurchmesser an der Oberseite größer ist als ein Außendurchmesser der Unterseite, um eine gute Handhabung bereitzustellen, und/oder wobei insbesondere ein Innendurchmesser an der Oberseite größer ist als ein Innendurchmesser der Unterseite, um eine gute Sichtbarkeit des Navigationsmarkers wie der Leuchtdiode zu gewährleisten. Durch die konische Form der (radialen) Außenflächen wird auch eine gute Handhabung geschaffen, da diese Form einen Hinterschnitt mit sich bringt. Auch kann, wenn die radiale Innenfläche, etwa ab Höhe einer Strahlungsebene de LED, ebenso eine konische Form aufweist, wird eine Sichtbarkeit (bei Befestigung an der LED) der emittierten Strahlung der LED verbessert, da eine trichterförmige Formgebung den Strahlungsbereich erweitert.

Insbesondere kann das Abdeckungsbefestigungselement eine konische Form aufweisen. Die konische Form des Abdeckungsbefestigungselements, insbesondere der Befestigungsschnittstelle des Abdeckungsbefestigungselements, reduziert die Belastung für die Abdeckfolie und ermöglicht zudem eine einfache Befestigung der LED-Abdeckung.

Gemäß einer weiteren Ausführungsform kann das Abdeckungsbefestigungselement in Richtung der Längsachse zu seiner Oberseite hin an seiner radialen Innenseite eine Stufe nach radial außen aufweisen, um im Bereich der Oberseite einen zu einer Längsachse hin versetzt beabstandeten Ring auszubilden, um ein Emittierungsfeld für insbesondere eine Leuchtdiode zu vergrößern. Es wird eine Art oder Form eines Tellers geschaffen, in dessen Mitte die Strahlungsquelle (überdeckt durch eine sterile Abdeckfolie) zu einer Seite hin eine weiter beabstandete Wand hat und ein Strahlungswinkel entsprechend vergrößert werden kann.

Vorzugsweise kann das Abdeckungsbefestigungselement selbst Polyoxymethylene (POM; thermoplastischer Kunststoff) als Material aufweisen oder gesamt aus diesem bestehen.

Insbesondere kann die Fixierung bzw. Befestigung der sterilen Abdeckfolie von der sterilen Seite her erfolgen. Hierfür kann das sterile Abdeckungsbefestigungselement von der sterilen Seite der sterilen Abdeckfolie her einfach auf den Navigationsmarker, insbesondere die Leuchtdiode, aufgeclipt (etwa mittels Schnappverschluss) und/oder magnetisch befestigt. Bei dieser Konfiguration kann beispielsweise eine übliche Abdeckfolie über einen chirurgischen Roboter, der die Navigationsmarker aufweist, geworfen werden, welche die Sterilbarriere ausbildet. Um nun einerseits die sterile Abdeckfolie an dem Roboter zu befestigen (dass diese nicht abfällt) als auch eine definierte Oberfläche über die Navigationsmarker zu gewährleisten, wird die sterile Abdeckfolie von der sterilen Seite her mit den sterilen Abdeckungsbefestigungselementen an den Navigationsmarkern befestigt. Hierdurch wird eine einfache und sichere Konfiguration geschaffen.

Vorzugsweise kann das Abdeckungsbefestigungselement eine dunkle Farbe haben, insbesondere selbst gesamt komplett schwarz gestaltet sein, um reflektierendes Licht bestmöglich zu absorbieren und zudem einen Kontrast zu dem Navigationsmarker, insbesondere der Leuchtdiode, zu erhöhen.

Insbesondere weist das Abdeckungsbefestigungselement an seiner Oberfläche eine dunkle Farbe, insbesondere Schwarz (bzw. ist in der Farbe schwarz), auf. Die schwarze Farbe hat den Vorteil, dass die Infrarot-LEDs aufgrund des höheren Kontrasts leichter zu erkennen sind.

Gemäß einer Ausführungsform kann das Abdeckungsbefestigungselement eine geriffelte radiale Außenseite aufweisen, um eine manuelle Handhabung zu verbessern. Insbesondere kann das Abdeckungsbefestigungselement eine Vielzahl von in Längsachse zueinander beabstandete umfängliche Riefen/Vertiefungen (als Nuten) aufweisen.

Insbesondere kann das Abdeckungsbefestigungselement als Schnappverbindung gestaltet sein und die Rastfinger einen Rastvorsprung nach radial außen aufweisen, um, wenn nach radial innen elastisch vorgespannt und an insbesondere der LED befestigt, in eine, insbesondere umfängliche, Innennut des LED-Gehäuses einzugreifen.

Insbesondere kann das Abdeckungsbefestigungselement rotationssymmetrisch gestaltet sein. Hierdurch ist keine spezielle Einsetzrichtung des Abdeckungsbefestigungselements gefordert und ein Nutzer kann das Abdeckungsbefestigungselement in beliebiger Ausrichtung um seine Längsachse aufsetzen und befestigen.

Vorzugsweise kann das Abdeckungsbefestigungselement nur abgerundete Kanten aufweisen, um die Abdeckfolie nicht zu beschädigen.

Insbesondere kann, in einem Längsschnitt entlang der Längsachse gesehen, das Abdeckungsbefestigungselement eine Konkave Querschnittsform oder eine L-förmige Querschnittsform aufweisen, etwa bei einer rotationssymmetrischen Gestaltung.

Hinsichtlich einer sterilen Abdeckfolie für eine lösbare Fixierung an einem Navigationsmarker, insbesondere einer Leuchtdiode, insbesondere an einer Leuchtdiode eines chirurgischen Roboters, wird die Aufgabe dadurch gelöst, dass an die sterile Abdeckfolie zumindest ein Abdeckungsbefestigungselement gemäß der vorliegenden Offenbarung starr fixiert/ unlösbar angebracht/befestigt sind und insbesondere die Abdeckfolie an der Oberseite des Abdeckungsbefestigungselements fixiert ist, sodass die sterile Abdeckfolie die Sterilbarriere nach außen hin bildet.

Neben einem System mit einer separaten sterilen Abdeckfolie und den Abdeckungsbefestigungselementen sind in dieser Ausführungsform der sterilen Abdeckfolie die Abdeckungsbefestigungselemente in die Folie selbst an den vorgesehenen Positionen integriert, so dass eine speziell angepasste sterile Abdeckfolie für eine Abdeckung etwa eines Roboters und eine Überdeckung der Navigationsmarker, insbesondere der Leuchtdioden, bereitgestellt wird.

Bei einer Variante eines integrierten magnetischen Abdeckungsbefestigungselements der sterilen Abdeckfolie ist das das Abdeckungsbefestigungselement mit Magnet fest auf der Innenseite der Abdeckfolie integriert. Diese Verbindung kann insbesondere durch Kleben, Folienschweißen und/oder einem Fügeverfahren hergestellt werden. Durch das Herstellen der magnetischen Verbindung wird die Folie über die LED gespannt und ermöglicht eine genaue Nachführung der LED.

Bei der Ausführungsform eines integrierten Snap-fit Elements bzw. eines integrierten Abdeckungsbefestigungselements mit Snap-fit-Funktion kann das Abdeckungsbefestigungselement fest in die Abdeckfolie integriert sein. Zur Fixierung des Abdeckungsbefestigungselements mit steriler Abdeckfolie an der LED-Befestigungsschnittstelle weist das Abdeckungsbefestigungselement eine Schnappgeometrie auf. Insbesondere kann diese durch die Verbindung der LED-Befestigungsschnittstelle und des Abdeckungsbefestigungselements die Abdeckfolie über die Tracking-LED gespannt werden.

Hinsichtlich eines Abdeckungsbefestigungssystem wird die Aufgabe dadurch gelöst, dass dieses einen Navigationsmarker, insbesondere eine Leuchtdiode, und ein Abdeckungsbefestigungselement gemäß der vorliegenden Offenbarung oder eine sterile Abdeckfolie gemäß der vorliegenden Offenbarung aufweist, und der Navigationsmarker, insbesondere die Leuchtdiode, eine LED-Befestigungsschnittstelle aufweist, wobei die LED-Befestigungsschnittstelle und die Abdeckungsbefestigungsschnittstelle aufeinander abgestimmte, zusammenwirkende komplementäre Koppelstrukturen ausbilden, sodass das Abdeckungsbefestigungselement lösbar kraftschlüssig und/oder formschlüssig an dem Navigationsmarker, insbesondere der Leuchtiode, befestigbar ist.

Insbesondere kann eine LED-Befestigungsschnittstelle und/oder Abdeckungsbefestigungsschnittstelle mit Hinterschneidung einen festen Sitz des sterilen Abdeckungsbefestigungselements (etwa LED-Abdeckung) bereitstellen und erlaubt gleichzeitig das Anbringen und Lösen des Abdeckungsbefestigungselements mit manueller Kraft. Es kann also eine temporäre (feste) Fixierung des Abdeckungsbefestigungselements erstellt werden, welche sich bei Bedarf manuell auch wieder lösen lässt, jedoch so gestaltet ist, dass es sich nicht unabsichtlich lösen lässt. Insbesondere kann das Abdeckungsbefestigungselement so ausgelegt sein, dass eine Kraft zum Lösen höher als ein festgelegter Grenzwert sein muss, um ein versehentliches Lösen zu unterbinden.

Insbesondere kann das Abdeckungsbefestigungssystem ein rotationssymmetrisches Trichtergehäuse aufweisen, in welches zentral das Gehäuse der Leuchtiode eingefügt ist.

Vorzugsweise kann der Navigationsmarker, insbesondere die Leuchtdiode, als LED-Befestigungsschnittstelle einen Ringmagneten aufweisen, der mit einem gegenpoligen Magneten, insbesondere einem Ringmagneten der Abdeckungsbefestigungsschnittstelle zusammenwirkt, um eine kraftschlüssige (komplementär gepolte) Befestigung bereitzustellen.

Gemäß einer Ausführungsform kann der Navigationsmarker, insbesondere die Leuchtdiode, ein hohlzylinderförmiges LED-Gehäuse entlang einer LED-Längsachse aufweisen und als LED-Befestigungsschnittstelle an der radialen Außenseite des LED-Gehäuses eine umfängliche umlaufende Nut aufweisen, um einen umfänglichen Hinterschnitt auszubilden, der mit den radialelastischen Rastfingern mit Rastvorsprung nach radial innen der Abdeckungsbefestigungsschnittstelle zusammenwirkt.

Gemäß einer weiteren Ausführungsform kann das Abdeckbefestigungselement als Befestigungsclip mit Schnappverbindung als Abdeckungsbefestigungsschnittstelle ausgebildet sein, wobei ein umfänglicher Spalt zwischen einem LED-Gehäuse des Navigationsmarkers, insbesondere der Leuchtdiode, und dem Abdeckungsbefestigungselement ausgebildet ist, der so gestaltet ist, dass eine sterile Abdeckfolie in diesen passgenau aufgenommen ist, insbesondere die Breite des Spalts der Dicke der Abdeckfolie entspricht oder geringfügig kleiner (beispielsweise 10% kleiner) ist, so dass die Abdeckfolie zwischen Abdeckungsbefestigungselement und Leuchtdiode (geometrisch passend) eingeklemmt ist.

Die Aufgaben werden hinsichtlich eines sterilen Abdeckungssystem gelöst durch: für einen chirurgischen Roboter mit einem an einer Roboterbasis angebundenen Roboterarm und einem an den Roboterarm angebundenen Roboterkopf (als oder mit Endeffektor), wobei der Roboterarm zumindest ein motorisiertes Roboterarmglied/- segment, insbesondere eine Vielzahl an aktiv aktuierbaren Roboterarmsegmenten aufweist, sowie eine Vielzahl an Navigationsmarkern, insbesondere Leuchtdioden (LED), aufweist, die an einer Oberfläche des zumindest einen Roboterarmsegments und/oder an dem Roboterkopf angeordnet sind, wobei diese Navigationsmarker, insbesondere Leuchtdioden, gegenüber der Oberfläche jeweils zumindest teilweise hervorstehen, wobei zumindest eine Teilmenge der Navigationsmarker, insbesondere Leuchtdioden, insbesondere alle, eine LED-Befestigungsschnittstelle umfasst, wobei das sterile Abdecksystem ferner umfasst: eine chirurgische Abdeckfolie, die so angepasst ist, dass es über die Navigationsmarker, insbesondere Leuchtdioden, angeordnet werden kann, welche sich auf der Oberfläche auf einem Roboterarmsegment und/oder dem Roboterkopf befinden, und eine Vielzahl von Abdeckungsbefestigungselementen gemäß der vorliegenden Offenbarung, die mit dem chirurgischen Abdecktuch zusammenwirken und lösbar über die Navigationsmarker, insbesondere Leuchtdioden, über deren LED-Befestigungsschnittstelle an- und abkoppelbar gestaltet sind, wobei jedes der Abdeckungsbefestigungselemente eine Öffnung für den Navigationsmarker, insbesondere die Leuchtdiode, aufweist, in welche der Navigationsmarker, insbesondere die Leuchtdiode, einführbar ist, wobei das Abdeckungsbefestigungselement eine komplementär zu der LED-Befestigungsschnittstelle ausgebildete Abdeckungsbefestigungsschnittstelle aufweist, welche mit der Befestigungsschnittstelle zusammenwirkt, um einen Teil der chirurgischen Abdeckungsfolie an den Navigationsmarkern, insbesondere Leuchtdioden zu sichern.

Insbesondere kann das sterile Abdeckungssystem als Set in einer sterilen Verpackung bereitgestellt werden, welche die sterile Abdeckfolie und die sterilen Abdeckungsbefestigungselemente aufweist.

Insbesondere kann das Abdeckungssystem dafür angepasst sein, dass die Fixierung bzw. Befestigung durch das Abdeckungsbefestigungselement von einer sterilen Seite her erfolgt.

Die Aufgaben werden hinsichtlich eines chirurgischer Roboters für einen chirurgischen Eingriff an einem Patienten, gelöst, dass dieser aufweist: einem an einer Roboterbasis angebundenen Roboterarm und einem an den Roboterarm angebundenen Roboterkopf (als oder mit Endeffektor), wobei der Roboterarm zumindest ein motorisiertes Roboterarmglied/-segment, insbesondere eine Vielzahl an aktiv aktuierbaren Roboterarmsegmenten aufweist, einer Vielzahl an Navigationsmarkern, insbesondere Leuchtdioden (LED), die an einer Oberfläche des zumindest einen Roboterarmsegments und/oder an dem Roboterkopf angeordnet sind, wobei diese Navigationsmarker, etwa Leuchtdioden, gegenüber der Oberfläche jeweils zumindest teilweise hervorstehen, wobei zumindest eine Teilmenge der Navigationsmarker, insbesondere Leuchtdioden, insbesondere alle, jeweils eine LED-Befestigungsschnittstelle umfasst, wobei der chirurgische Roboter ferner aufweist: zumindest ein Abdeckungsbefestigungselement oder eine sterile Abdeckfolie oder ein Abdeckungsbefestigungssystem oder ein steriles Abdeckungssystem jeweils gemäß der vorliegenden Offenbarung.

Insbesondere hat also das Abdeckungsbefestigungselement im Bereich seiner Oberseite (also an seinem oberen Ende) eine Hinterschneidung/ einen Hinterschnitt nach radial außen, die zum manuellen Abnehmen des Abdeckungsbefestigungselements geeignet ist. So kann ein manuelles Ergreifen des Abdeckungsbefestigungselements erleichtert und etwa ein Abziehen

Das Abdeckungsbefestigungselement weist insbesondere acht einrastbare Rastfinger/Cliparme/Rastarme auf.

Es kann vorteilhaft sein, die LED-Befestigungsschnittstelle und/oder die Abdeckungsbefestigungsschnittstelle in schwarzer Farbe zu gestalten, um den Kontrast zur LED zu erhöhen.

Die Aufgaben werden hinsichtlich eines Herstellungsverfahrens einer sterilen Abdeckfolie dadurch gelöst, dass dieses die Schritte aufweist: Anordnen eines Abdeckungsbefestigungselements an einer sterilen Abdeckfolie; und
Fügen des Abdeckbefestigungselements mit der Abdeckfolie, insbesondere durch Kleben oder Folienschweißen.

Alle Offenbarungen hinsichtlich des Abdeckungsbefestigungselements gemäß der vorliegenden Offenbarung gelten ebenso hinsichtlich der sterilen Abdeckfolie, des Abdeckungsbefestigungssystems, des Abdeckungssystems und des chirurgischen Roboters der vorliegenden Offenbarung.

### Kurzbeschreibung der Figuren

Die vorliegende Offenbarung wird nachfolgend anhand bevorzugter Ausführungsformen unter Bezugnahme auf die begleitenden Figuren näher erläutert. Es zeigen:
Fig. 1 eine perspektivische Ansicht eines Abdeckungsbefestigungselements gemäß einer bevorzugten Ausführungsform, das in einem Abdeckungsbefestigungssystem einer bevorzugten Ausführungsform eingesetzt wird;
Fig. 2 eine Längsschnittansicht durch das Abdeckungsbefestigungssystem aus Fig. 1, wobei eine transparente sterile Abdeckfolie zwischen der **LED** und dem Abdeckungsbefestigungselement angeordnet jedoch noch nicht befestigt ist;
Fig. 3 eine weitere Längsschnittansicht wie Fig. 2, wobei jedoch vorliegend das Abdeckungsbefestigungselement an der **LED** befestigt ist und die sterile Abdeckfolie mit befestigt;
Fig. 4 eine perspektivische Ansicht des Zustands des Abdeckungsbefestigungssystems wie in Fig. 3 gezeigt.
Fig. 5 eine perspektivische Ansicht eines chirurgischen Roboters einer bevorzugten Ausführungsform der vorliegenden Offenbarung mit dem Abdeckungsbefestigungssystem und Abdeckungsbefestigungselement aus Fign. 1 bis 4;
Fig. 6 eine Längsschnittansicht eines Abdeckungsbefestigungssystems einer weiteren bevorzugten Ausführungsform, bei der das Abdeckungsbefestigungselement einer weiteren bevorzugten Ausführungsform einen Magneten für eine Befestigung an einer **LED** mit Magnet aufweist, wobei das Abdeckungsbefestigungselement noch nicht befestigt ist;
Fig. 7 eine weitere Längsschnittansicht wie Fig. 6, nur mit dem Unterschied, dass das Abdeckungsbefestigungselement an der **LED** befestigt ist und die sterile Abdeckfolie spannt und einklemmt;
Fig. 8 eine perspektivische Ansicht des Abdeckungsbefestigungssystems aus Fig. 7;
Fig. 9 eine perspektivische Ansicht des Abdeckungsbefestigungselements aus Fign. 6 bis 8;
Fig. 10 eine perspektivische Teilansicht eines chirurgischen Roboters gemäß einer weiteren bevorzugten Ausführungsform, an dessen Roboterkopf an die LEDs die sterile Abdeckfolie und die Abdeckungsbefestigungselemente angebracht sind;
Fig. 11 eine perspektivische Teilansicht einer sterilen Abdeckfolie einer weiteren bevorzugten Ausführungsform, welche fest integrierte Abdeckungsbefestigungselemente aufweist;
Fig. 12 eine Längsschnittansicht eines Abdeckungssystems einer bevorzugten Ausführungsform mit der sterilen Abdeckfolie mit integrierten Abdeckungsbefestigungselementen aus Fig. 11;
Fig. 13 eine weitere Längsschnittansicht wie Fig. 12 nur mit dem Unterschied, dass die sterile Abdeckfolie an der LED über Magneten befestigt ist;
Fign. 14 & 15 weitere perspektivische Ansichten des Systems aus Fign 11 bis 13;
Fig. 16 eine perspektivische Vertiefung bzw. hervorstehen einer LED aus einer Oberfläche als Andockbereich für ein Abdeckungsbefestigungselement gemäß einer weiteren bevorzugten Ausführungsform;
Fign. 17 & 18 eine Längsschnittansicht und eine perspektivische Ansicht eines Abdeckungsbefestigungselements, das mit dem Andockbereich aus Fig. 16 zusammenwirkt und das Rastvorsprünge nach radial nach außen aufweist;
Fign 19 und 20 jeweils eine perspektivische Ansicht einer sterilen Abdeckfolie, welche integrierte Abdeckungsbefestigungselemente mit einer Schnappverbindung aufweist.

Die Figuren sind schematischer Natur und sollen nur dem Verständnis der Erfindung dienen. Gleiche Elemente sind mit denselben Bezugszeichen versehen. Die Merkmale der verschiedenen Ausführungsformen können untereinander ausgetauscht werden.

### Detaillierte Beschreibung bevorzugter Ausführungsformen

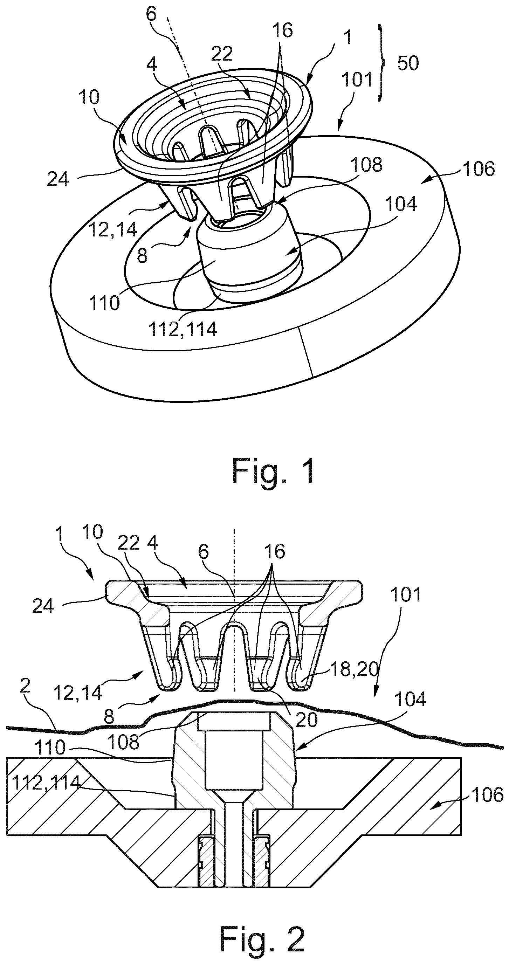

Fign. 1 bis 5 zeigen ein Abdeckungsbefestigungselement, ein Abdeckungsbefestigungssystem, ein Abdecksystem sowie einen chirurgischen Roboter jeweils gemäß einer ersten bevorzugten Ausführungsform der vorliegenden Offenbarung, die miteinander zusammenwirken bzw. Teilkomponenten voneinander bilden.

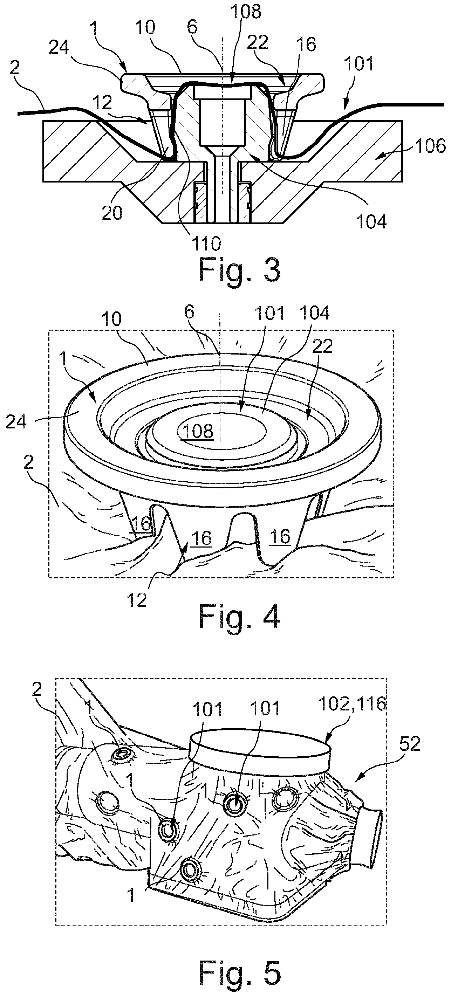

Fig. 1 zeigt in einer perspektivischen Ansicht ein medizinisches Abdeckungsbefestigungselement 1 für eine lösbare Fixierung einer sterilen Abdeckfolie 2 (siehe Fig. 2) an einer Leuchtdiode 101 (als Navigationsmarker) eines chirurgischen Roboters 102 (siehe Fig. 5). Wie in Fig. 1 ersichtlich, weist das Abdeckungsbefestigungselement 1 eine zentrale durchgängige Öffnung 4 als ein zentrales kreisförmiges Loch auf, wobei sich die Öffnung 4 entlang einer Längsachse 6 erstreckt, zwischen einer Unterseite 6 und einer Oberseite 10 des Abdeckungsbefestigungselements 1.

Durch diese Öffnung 4 entlang der Längsachse 6 kann von der Unterseite 8 her eine vorstehende Leuchtdiode 101 (mit LED-Gehäuse) in Richtung der Oberseite (10) eingeführt werden.

Das Abdeckungsbefestigungselement 1 ist für den chirurgischen Einsatz steril und sterilisierbar ausgebildet. Insbesondere kann das Abdeckungsbefestigungselement 1 ein sterilisierbares Material aufweisen.

Das Abdeckungsbefestigungselement 1 weist an bzw. im Bereich seiner Unterseite 8 eine Abdeckungsbefestigungsschnittstelle 12 auf, über welche das Abdeckungsbefestigungselement 1 lösbar kraftschlüssig als auch formschlüssig befestigbar ist, wie nachstehend erläutert.

Konkret ist in dieser Ausführungsform die Abdeckungsbefestigungsschnittstelle 12 als Schnappverbindung 14 ausgestaltet, welche über acht entlang der Längsachse zur Unterseite hin freischwingende und abstehende Rastfinger 16 aufweist, die umfänglich gleichverteilt angeordnet sind.

Diese Rastfinger 16 bilden selbst die Unterseite 8 des Abdeckungsbefestigungselements 1 auf und sind leicht konisch, also trichterförmig angeordnet. Dies begünstigt, wie später erklärt, eine Handhabung und ein Einschnappen. Ferner sind die Rastfinger 16 alle gleich ausgebildet und verjüngen sich in ihrer Breite entlang der Längsachse 6 zu ihrer Rastspitze 18 hin. An der Rastspitze 18 ist jeweils zudem ein nach radial innen gerichteter Rastvorsprung 20 ausgebildet.

Im Bereich der Oberseite 10 ist an der radialen Innenseite eine umfängliche Stufe 22 ausgebildet, welche einen ringförmigen Kragen 24 des Abdeckungsbefestigungselements 1 nach radial außen ausbildet. Dieser Kragen 24 dient einer besonders guten Handhabung des Abdeckungsbefestigungselements 1 und zudem einer Erweiterung eines möglichen Strahlungsbereichs.

Wie in Fig. 1 weiter dargestellt ist die zu dem Abdeckungsbefestigungselement 1 passende (standardisierter) Leuchtdiode 101 mit einem inneren LED-Gehäuse 104 und einem trichterförmigen LED-Teller 106 ausgestattet. Natürlich hat die Leuchtdiode 101 mittig im Zentrum im LED-Gehäuse 104 eine lichtemittierende Strahlungsquelle (eine Diode), um nachverfolgt zu werden.

Das LED-Gehäuse weist eine im Wesentlichen zylinderförmige Grundform auf mit einer planen LED-Oberseite 108 (aus welcher die Strahlung emittiert wird) wobei ein Übergang von Seitenwand zu Oberseite abgerundet gestaltet ist (ähnlich eines Kopfes eines Legomännchens). Im Bereich des Kontaktes von LED-Gehäuse 104 und LED-Teller 6 ist an der radialen Außenseite 110 eine umfängliche Nut 112 eingebracht, welche einen Hinterschnitt für die Leuchtdiode 101 bildet. Diese Nut 112 dient, wie später zu den Fign 2 bis 4 beschrieben, der Befestigung des Abdeckungsbefestigungselements 1 und bildet eine zu der Abdeckungsbefestigungsschnittstelle12 komplementäre LED-Befestigungsschnittstelle 114. Der LED-Teller 106 wiederum hat eine schwarze Oberfläche und begünstigt einen Kontrast zur Leuchtiode 101 und damit einer Nachverfolgung. Auch wird durch die Tellerform eine Art weicher Übergang geschaffen.

Das Abdeckungsbefestigungselement 1 und die Leichtdiode 101 bilden zusammen das Abdeckungsbefestigungssystem 50 einer bevorzugten Ausführungsform der vorliegenden Offenbarung. Hierbei hat die Leuchtdiode 101 die LED-Befestigungsschnittstelle 114 und die LED-Befestigungsschnittstelle 114 sowie die Abdeckungsbefestigungsschnittstelle 12 sind so aufeinander abgestimmt und wirken so zusammen, dass sie komplementäre Koppelstrukturen ausbilden, sodass das Abdeckungsbefestigungselement lösbar kraftschlüssig und/oder formschlüssig an der Leuchtiode befestigbar ist. Der Begriff Koppelstruktur ist nicht nur geometrisch zu sehen, sondern beinhaltet auch eine Funktionsstruktur für eine Kopplung über einen Magneten oder eine magnetisierbare Struktur (Elektromagneten).

Fig.2 zeigt einen Längsschnitt des Abdeckungsbefestigungselements 1 aus Fig. 1, wobei jedoch zwischen dem Abdeckungsbefestigungselement 1 und der Leuchtdiode 101 die sterile Abdeckfolie / das sterile Abdecktuch 2 zwischen angeordnet ist. Wie auch in Fig. 1 zeigt Fig. 2 den nicht befestigten Zustand. Wird nun das Abdeckungsbefestigungselement 1 entlang seiner Längsachse 6 zu seiner Unterseite 8 hin an die Leuchtdiode geführt, so ergibt sich bei Anschlag an dem LED-Teller 6 der in Fig. 3 gezeigte Zustand.

Fig. 3 zeigt nämlich den Zustand des Abdeckungsbefestigungssystems 50 bzw. des Abdeckungsbefestigungselements 1, bei dem dieses formschlüssig und kraftschlüssig auf der Leuchtdiode 101 aufsitzt und zwischen der Leuchtdiode 101 und dem Abdeckungsbefestigungselement 1 die sterile Abdeckfolie 2 eingeklemmt ist. Durch die Rastfinger 16 wird beim Absenken von Fig. 2 zu Fig. 3 zunächst an einem oberen Bereich des LED-Gehäuses 104 die sterile Abdeckfolie 2 angelegt und durch die radialelastische Vorspannung der Rastfinder 16 über die LED-Oberseite 108 gespannt. Bei weiterem Absenken des Abdeckungsbefestigungselements 1 wird die Abdeckfolie 2 zwangsläufig über die Rastfinger 16 mit nach unten gezogen und über die LED-Oberseite weiter gespannt. In der in Fig. 3 gezeigten Endposition des Abdeckungsbefestigungselements 1 greifen alle Rastfinger 16 umfänglich homogen in die Nut 112 ein und die Rastfinger schnappen zumindest teilweise entgegen ihrer Vorspannung zurück in Richtung ihrer statischen Position ohne Krafteinwirkung. Dadurch ergibt sich ein Formschluss und zudem ein Kraftschluss und die Abdeckfolie wird sicher gehalten und befestigt und liegt perfekt gespannt über der Strahlungsöffnung über welche die Strahlung emittiert wird. Durch die vordefinierte Lage der gespannten Abdeckfolie 2 können Reflexionen verhindert werden und eine standardisierte sterile Abdeckung für die Leuchtdioden 101 geschaffen werden.

Durch den LED-Teller 106 wird die Abdeckfolie 2 zu den Seiten hin über eine Art Böschung wieder zu der "normalen" Oberfläche hin geführt und kann den chirurgischen Roboter 102 weiter abdecken.

Fig. 4 zeigt eine perspektivische Ansicht des Abdeckungsbefestigungssystems 50 mit Abdeckungsbefestigungselement 1, welches die Abdeckfolie 2 straff über die Leuchtdiode 101 spannt.

Fig. 5 zeigt wiederum eine perspektivische Ansicht des chirurgischen Roboters 102 einer ersten bevorzugten Ausführungsform, der an seinem Roboterkopf 116 die Abdeckungsbefestigungselemente 1 zur Befestigung der Abdeckfolie 2 an den Leuchtdioden 101 aufweist.

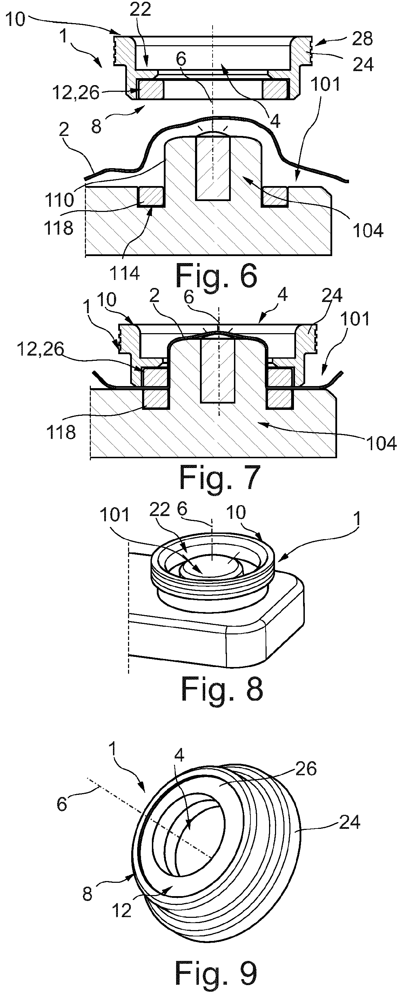

Fign. 6 bis 10 zeigen eine weitere, jeweils zweite bevorzugte Ausführungsform eines Abdeckungsbefestigungselements 1, eines Abdeckungsbefestigungssystem 50, ein Abdecksystem 52 sowie einen chirurgischen Roboter 2.

Im Unterschied zu der vorangegangenen Ausführungsform(en) wird keine geometrische Schnappverbindung verwendet, sondern eine magnetische Kopplung und damit Befestigung.

Konkret zeigt Fig. 6 ein Abdeckungsbefestigungselement 1 mit einem ringförmigen Magneten 26 an seiner Unterseite 8. Die Leuchtdiode 101 andererseits weist ebenfalls einen ringförmigen Magneten 118 auf. Zwischen der Leuchtdiode 101 und dem Abdeckungsbefestigungselement 1 ist in dem nicht befestigten Zustand wieder die Abdeckfolie 2 angeordnet.

Wird nun, analog zu dem Vorgehen der ersten Ausführungsform, das Abdeckungsbefestigungselement 1 abgesenkt, so wird das Abdeckungsbefestigungselement 1 an der Leuchtdiode befestigt, wie in Fig. 7 gezeigt. Konkret ist zwischen der radialen Außenseite 110 des LED-Gehäuses 8 und dem Magneten 26, der die Abdeckungsbefestigungsschnittstelle 12 bildet, ein solcher Spalt vorgesehen, dass genau die Abdeckfolie hineinpasst und durch ganz leichte Presspassung nach unten gezogen wird. So wird über die Strahlungsquelle der Leuchtdiode 101 die Abdeckfolie 2 gestrafft und bildet eine Sterilbarriere aus. Die Leuchtdiode 101 selbst muss nicht steril sein.

Das Abdeckungsbefestigungselement 1 weist eine geriffelte radiale Außenstruktur 28 auf, um eine Handhabung wie etwa ein Abziehen des Abdeckungsbefestigungselements 1 von der Leuchtdiode 101 zu vereinfachen. Auch ist wieder eine Stufe 22 ausgebildet, welche den Kragen 24 aufweitet und einen Winkel einer möglichen Abstrahlung der Strahlungsquelle vergrößert.

Fig. 8 und 9 zeigen das Abdeckungsbefestigungselement 1, in welches an seiner Unterseite der ringförmige Magnet eingesetzt ist. In dieser Ausführungsform ist kein LED-Teller 106 vorgesehen, sondern eine plane Oberfläche, auf welche sich die Abdeckfolie 2 legt.

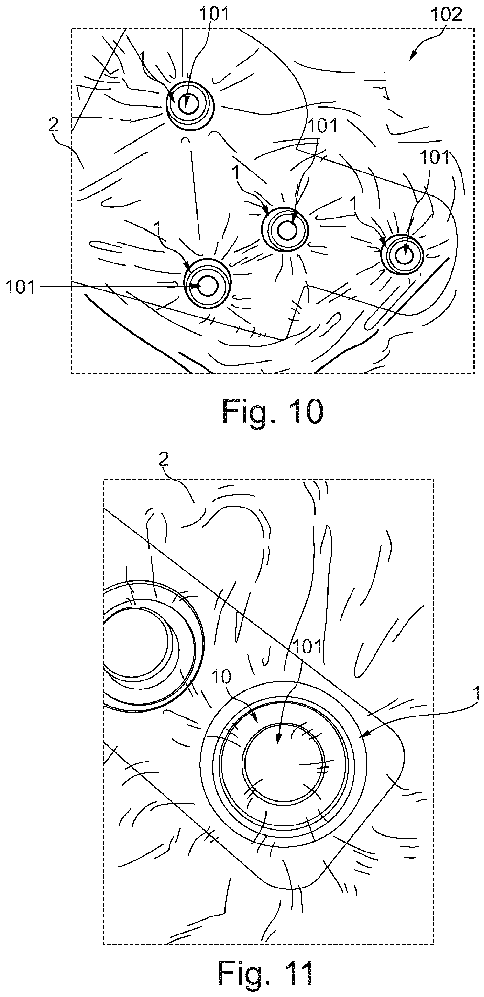

Fig. 10 zeigt in einer perspektivischen Teilansicht einen chirurgischen Roboter 102 einer weiteren bevorzugten Ausführungsform, an welchen die Abdeckungsbefestigungselemente 1 der Fign. 6 bis 9 magnetisch gehalten befestigt sind und die Abdeckfolie 2 über die Strahlungsquelle spannen.

Fign. 11 bis 15 zeigen eine sterile Abdeckfolie 2 gemäß einer bevorzugten Ausführungsform, welche integrierte Abdeckungsbefestigungselemente 1 aufweist. Integriert meint, dass die Abdeckungsbefestigungselemente 1 an vorbestimmten Positionen der Abdeckfolie 2 fest an diese angebunden sind, insbesondere durch Kleben, Folienschweißen und/oder einem anderen Fügeverfahren starr fixiert sind. Durch die Herstellung

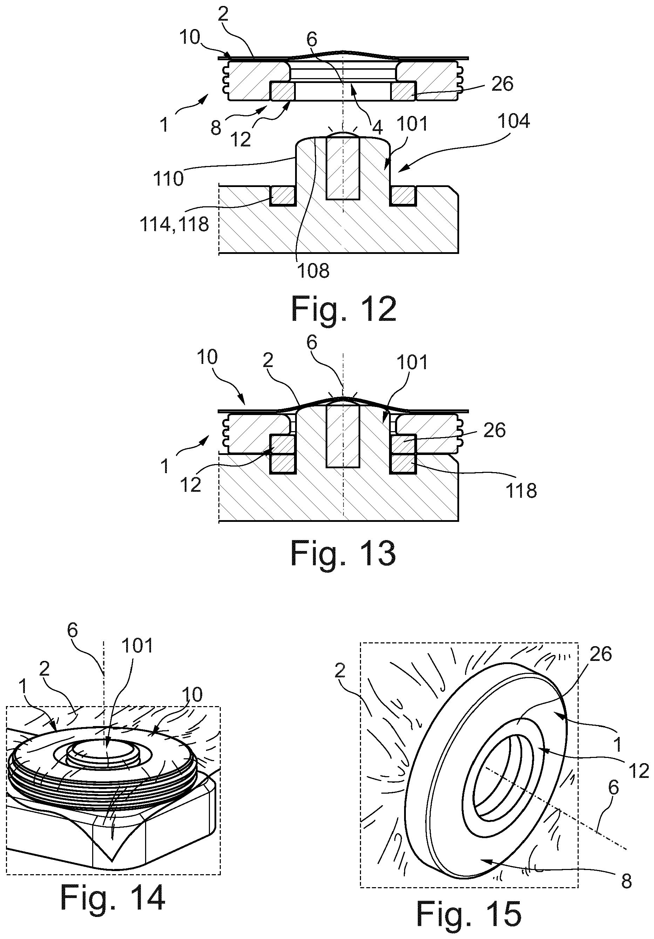

Fig. 12 zeigt in einer Längsschnittansicht, wie das an die Abdeckfolie 2 gefügte Abdeckungsbefestigungselement 1 im Bereich des Abdeckungsbefestigungselements 1 eine gespannte Oberfläche der Abdeckfolie 2 erzeugt und bei einem Aufsetzen auf die Leuchtdiode 101, wie in Fig. 13 gezeigt, eine definierte Oberfläche einer Sterilbarriere bereitstellt.

Fig. 14 und 15 zeigen weitere Ansichten der sterilen Abdeckfolie 2 mit den Abdeckbefestigungselementen 1 (des Abdeckungsbefestigungssystems 50).

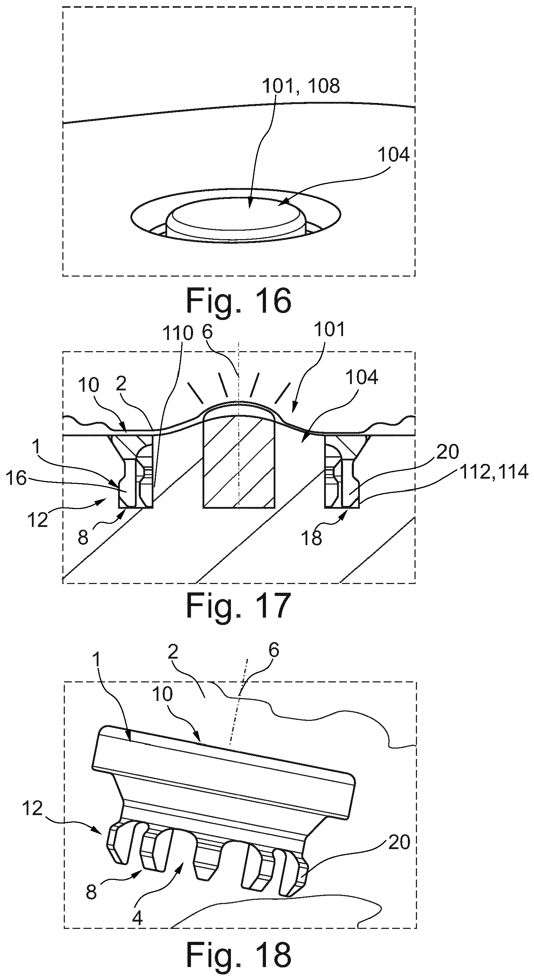

Fign. 16 bis 18 zeigen eine weitere Ausführungsform eines Abdeckungsbefestigungselements 1, welches (ähnlich der ersten Ausführungsform) eine Schnappverbindung mit Rastfingern 16 hat, welche jedoch in dieser Ausführungsform nicht Rastvorsprünge 20 nach radial innen aufweisen, sondern nach radial außen und sich an eine radiale Innenfläche einer Umgebung der Leuchtdiode 101 anpressen. In dieser Ausführungsform ist in einer Wand um das hervorstehende LED-Gehäuse 104 herum, eine umfängliche (Innen-)Nut 112 vorgesehen, welche sich konzentrisch um das LED-Gehäuse 104 herum erstreckt. Die Rastfinger werden nun bei einem Einsetzen nun nach radial innen vorgespannt und rasten, durch ihre elastische Vorspannkraft nach radial außen in die Nut 112 entsprechend ein.

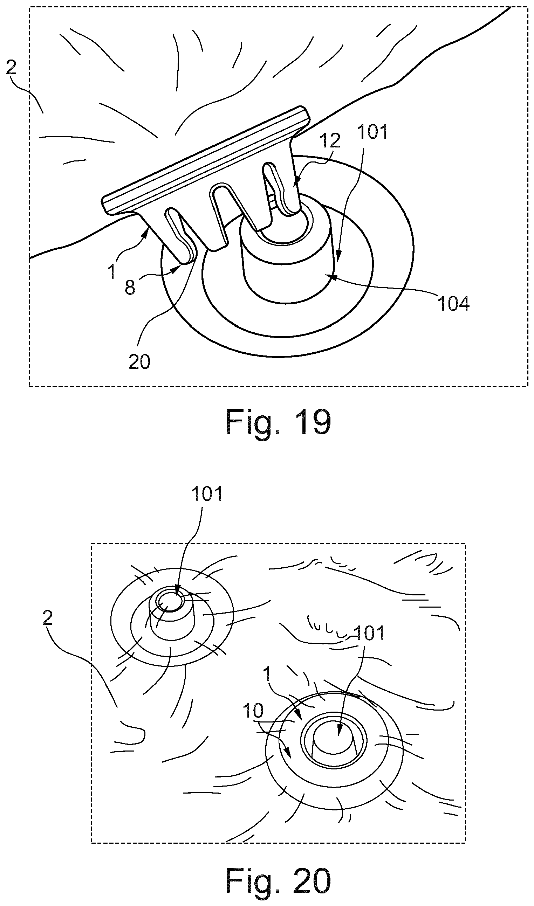

Fign. 19 und 20 zeigen eine weitere bevorzugte Ausführungsform der sterilen Abdeckfolie 2, welche integrierte Abdeckungsbefestigungselemente 1 aufweist. Im Unterschied zu der ersten Ausführungsform sind vorliegend die Abdeckungsbefestigungselemente 1 als Schnappverbindung ausgebildet und im Wesentlichen gleich zu dem Abdeckungsbefestigungselement 1 aus Fig. 1 gestaltet. Die sterile Abdeckfolie 2 ist an der Oberseite 10 an das Abdeckungsbefestigungselement 1 gefügt, hier foliengeschweißt.

### Bezugszeichenliste

- 1: Abdeckungsbefestigungselement
- 2: Abdeckfolie
- 4: Öffnung
- 6: Längsachse
- 8: Unterseite
- 10: Oberseite
- 12: Abdeckungsbefestigungsschnittstelle
- 14: Schnappverbindung
- 16: Rastfinger
- 18: Rastspitze
- 20: Rastvorsprung
- 22: Stufe
- 24: Kragen
- 26: Magnet
- 28: geriffelte (Handhabungs-)Struktur

- 50: Abdeckungsbefestigungssystem
- 52: Abdecksystem

- 101: Leuchtdiode/LED (als Navigationsmarker)
- 102: chirurgischer Roboter
- 104: LED-Gehäuse
- 106: LED-Teller
- 108: LED-Oberseite
- 110: Außenseite
- 112: Nut
- 114: LED-Befestigungsschnittstelle
- 116: Roboterkopf
- 118: Magnet

## Patentansprüche

1. Chirurgischer Roboter (102) für einen chirurgischen Eingriff an einem Patienten, mit:
einem an einer Roboterbasis angebundenen Roboterarm und einem an den Roboterarm angebundenen Roboterkopf (116), wobei der Roboterarm zumindest ein motorisiertes Roboterarmsegment aufweist,
einer Vielzahl an Navigationsmarkern, die an einer Oberfläche des zumindest einen Roboterarmsegments und/oder an dem Roboterkopf (116) angeordnet sind, wobei diese Navigationsmarker gegenüber der Oberfläche jeweils zumindest teilweise hervorstehen, wobei zumindest eine Teilmenge der Navigationsmarker eine LED-Befestigungsschnittstelle (114) umfasst,
eine chirurgische sterile Abdeckfolie (2), die so angepasst ist, dass sie über die Navigationsmarker angeordnet werden kann, und
eine Vielzahl von Abdeckungsbefestigungselementen (1), wobei die Abdeckungsbefestigungselemente (1) mit der chirurgischen sterilen Abdeckfolie (2) zusammenwirken und lösbar über die Navigationsmarker über deren LED-Befestigungsschnittstelle (114) an- und abkoppelbar gestaltet sind, wobei jedes der Abdeckungsbefestigungselemente (1) eine Öffnung (4) entlang einer Längsachse (6) von einer Unterseite (8) zu einer Oberseite (10) des Abdeckungsbefestigungselements (1) aufweist, in welche der Navigationsmarker einführbar ist,
**dadurch gekennzeichnet, dass**
jedes der Abdeckungsbefestigungselemente (1) eine komplementär zu der LED-Befestigungsschnittstelle (114) ausgebildete Abdeckungsbefestigungsschnittstelle (12) im Bereich der Unterseite (8) des Abdeckbefestigungselements (1) aufweist, über welche das Abdeckungsbefestigungselement (1) lösbar kraftschlüssig und/oder formschlüssig befestigbar ist und welche mit der LED-Befestigungsschnittstelle (114) zusammenwirkt, um einen Teil der chirurgischen sterilen Abdeckfolie (2) an den Navigationsmarkern zu befestigen und zu sichern.

2. Chirurgischer Roboter (102) nach Anspruch 1, **dadurch gekennzeichnet, dass** das Abdeckbefestigungselement (1) als Befestigungsclip mit Schnappverbindung (14) als Abdeckungsbefestigungsschnittstelle (12) ausgebildet ist, bei dem sich zumindest ein, insbesondere mehrere, Rastfinger (16) in Richtung der Längsachse (6) zu der Unterseite (8) hin erstreckt und in radialer Richtung elastisch auslenkbar ist, um das Abdeckungsbefestigungselement (1) kraftschlüssig und/oder formschlüssig zu halten.

3. Chirurgischer Roboter (102) nach Anspruch 2, **dadurch gekennzeichnet, dass** der Rastfinger (16) für eine Ausbildung eines Hinterschnitts für einen Formschluss einen endständigen Rastvorsprung (20) nach radial innen aufweist.

4. Chirurgischer Roboter (102) nach Anspruch 2 oder 3, **dadurch gekennzeichnet, dass** das Abdeckbefestigungselement (1) um seine Öffnung herum gleichverteilte, insbesondere rotationssymmetrisch angeordnete, zu seiner Unterseite (8) hin vorstehende Rastfinger (16) aufweist, welche eine homogene umfängliche Fixierung des Abdeckungsbefestigungselements (1) gewährleisten und die Rastfinger (16) insbesondere selbst die Unterseite (8) ausbilden.

5. Chirurgischer Roboter (102) nach einem der Ansprüche 1 bis 4, **dadurch gekennzeichnet, dass** der Navigationsmarker ein hohlzylinderförmiges LED-Gehäuse (104) entlang einer LED-Längsachse aufweist und als LED-Befestigungsschnittstelle (114) an der radialen Außenseite des LED-Gehäuses (114) eine umfängliche umlaufende Nut (112) aufweist, um einen umfänglichen Hinterschnitt auszubilden, der mit den radialelastischen Rastfingern (16) mit Rastvorsprung (20) nach radial innen der Abdeckungsbefestigungsschnittstelle (12) zusammenwirkt.

6. Chirurgischer Roboter (102) nach einem der Ansprüche 1 bis 5, **dadurch gekennzeichnet, dass** das Abdeckbefestigungselement (1) als Befestigungsclip mit Schnappverbindung (14) als Abdeckungsbefestigungsschnittstelle (12) ausgebildet ist, wobei ein umfänglicher Spalt zwischen einem LED-Gehäuse (114) des Navigationsmarkers und dem Abdeckungsbefestigungselement (1) ausgebildet ist, der so gestaltet ist, dass eine sterile Abdeckfolie (2) in diesen passgenau aufgenommen ist, insbesondere die Breite des Spalts der Dicke der Abdeckfolie (2) entspricht, so dass die Abdeckfolie (2) zwischen Abdeckungsbefestigungselement (1) und Navigationsmarker, insbesondere Leuchtdiode (101) eingeklemmt ist.

7. Chirurgischer Roboter (102) nach einem der Ansprüche 1 bis 6, **dadurch gekennzeichnet, dass** die Abdeckfolie (2) an der Oberseite (10) des Abdeckungsbefestigungselements (1) starr fixiert ist, sodass die sterile Abdeckfolie (2) die Sterilbarriere nach außen hin bildet.

8. Chirurgischer Roboter (102) nach einem der Ansprüche 1 bis 7, **dadurch gekennzeichnet, dass** das Abdeckungsbefestigungselement (1) im Bereich seiner Oberseite (10) zumindest einen, insbesondere umfänglichen, Vorsprung nach radial außen aufweist, um einen Hinterschnitt für eine manuelle Handhabung auszubilden.

9. Chirurgischer Roboter (102) nach einem der Ansprüche 1 bis 8, **dadurch gekennzeichnet, dass** das Abdeckungsbefestigungselement (1) rotationssymmetrisch gestaltet ist und in Richtung der Längsachse (6) insbesondere eine konische Formgebung aufweist,
wobei insbesondere ein Außendurchmesser an der Oberseite (10) größer ist als ein Außendurchmesser der Unterseite (8), um eine gute Handhabung bereitzustellen, und/oder
wobei insbesondere ein Innendurchmesser an der Oberseite (10) größer ist als ein Innendurchmesser der Unterseite (8), um eine gute Sichtbarkeit des Navigationsmarkers zu gewährleisten.

10. Chirurgischer Roboter (102) nach einem der Ansprüche 1 bis 9, **dadurch gekennzeichnet, dass** das Abdeckungsbefestigungselement (1) in Richtung der Längsachse (6) zu seiner Oberseite (10) hin an seiner radialen Innenseite eine Stufe (22) nach radial außen aufweist, um im Bereich der Oberseite (10) einen zu einer Längsachse (6) hin versetzt beabstandeten Ring auszubilden, um ein Emittierungsfeld für eine Strahlung zu vergrößern.

11. Chirurgischer Roboter (102) nach einem der Ansprüche 1 bis 10, **dadurch gekennzeichnet, dass** das Abdeckungsbefestigungselement (1) als Material Polyoxymethylene als Material aufweist, insbesondere gesamt aus diesem besteht, und vorzugsweise eine dunkle Farbe hat, um reflektierendes Licht bestmöglich zu absorbieren und zudem einen Kontrast zu dem Navigationsmarker zu erhöhen.

## Claims

1. A surgical robot (102) for a surgical procedure on a patient, comprising:
a robot arm attached to a robot base and a robot head (116) attached to the robot arm, the robot arm having at least one motorized robot arm segment,
a plurality of navigation markers, which are arranged on a surface of the at least one robot arm segment and/or on the robot head (116), wherein these navigation markers, in each case at least partially protrude with respect to the surface, wherein at least a partial quantity of the navigation markers, comprise an LED fastening interface (114),
a surgical sterile cover film (2) adapted to be placed over the navigation markers, and
a plurality of cover fastening elements (1), wherein the cover fastening elements (1) interact with the surgical sterile cover film (2) and are designed such that they can be releasably coupled to and uncoupled from the navigation markers via the LED fastening interface (114), wherein each of the cover fastening elements (1) comprises an opening (4) along a longitudinal axis from an underside (8) to an upper side (10) of the cover fastening element (1), wherein the navigation marker can be introduced into the opening (4),
**characterized in that**
each of the cover fastening elements (1) comprises a cover fastening interface (2) in the region of the underside (8) of the cover fastening element (1), wherein the cover fastening interface (2) is formed so as to be complementary to the LED fastening interface (114), wherein the cover fastening element (1) can be releasably fastened force-fittingly and/or form-fittingly via the cover fastening interface (2), and wherein the cover fastening interface (2) is interacting with the LED fastening interface (114) in order to fasten and secure a part of the surgical cover film (2) to the navigation markers.

2. The surgical robot (102) according to claim 1, **characterized in that** the cover fastening element (1) is formed as a fastening clip with snap connection (14) as cover fastening interface (12), in which at least one, in particular a plurality of latching finger(s) (16) extend(s) in the direction of the longitudinal axis (6) towards the underside (8), and can be elastically deflected in the radial direction in order to hold the cover fastening element (1) force-fittingly and/or form-fittingly.

3. The surgical robot (102) according to claim 2, **characterized in that** the latching finger (16) comprises an end latching protrusion (20) facing radially inwards for forming an undercut for form fit.

4. The surgical robot (102) according to claim 2 or 3, **characterized in that** the cover fastening element (1) comprises, around its opening (4), evenly distributed, in particular rotationally symmetrically arranged, latching fingers (16) protruding towards its underside (8), which ensure homogeneous peripheral fixing of the cover fastening element (1), and form the latching fingers (16) themselves, in particular the underside (8).

5. The surgical robot (102) according to one of claims 1 to 4, **characterized in that** the navigation marker comprises a hollow-cylindrical LED housing (104) along a longitudinal LED axis, and, as an LED fastening interface (114) on the radial outer side of the LED housing (114), comprises a circumferential groove (112) to form a circumferential undercut which interacts with the radially elastic latching fingers (16) with latching protrusions (20) in the radially inward direction of the cover fastening interface (12).

6. The surgical robot (102) according to one of claims 1 to 5, **characterized in that** the cover fastening element (1) is configured as a fastening clip with a snap connection as cover fastening interface (2), wherein a circumferential gap is formed between an LED housing (114) of the navigation marker, and the cover fastening element (1), which is configured such that a sterile cover film (2) is accommodated therein with an exact fit, and that in particular the width of the gap corresponds to the thickness of the cover film (2), so that the cover film (2) is clamped between the cover fastening element (1) and the navigation marker, in particular the light emitting diode (101).

7. The surgical robot (102) according to one of claims 1 to 6, **characterized in that** the cover film (2) is fixed to the upper side (10) of the cover fastening element (1), so that the sterile cover film (2) forms a sterile barrier to the outside.

8. The surgical robot (102) according to claim 1, **characterized in that** the cover fastening element (1) comprises, in the region of its upper side (10), at least one, in particular peripheral, protrusion radially outwards, in order to form an undercut for manual handling.

9. The surgical robot (102) according to one of claims 1 to 8, **characterized in that** the cover fastening element (1) is designed rotationally symmetrical and has, in particular, a conical shape in the direction of the longitudinal axis (6),
wherein in particular an external diameter at the upper side (10) is larger than an external diameter of the underside (8) in order to provide good handling, and/or
wherein in particular an inner diameter at the upper side (10) is larger than an inner diameter of the underside (8) in order to ensure good visibility of the navigation marker.

10. The surgical robot (102) according to one of the claims 1 to 9,
**characterized in that** the cover fastening element (1) comprises, in the direction of the longitudinal axis (6) towards its upper side (10), on its radially inner side, a step (22) towards the radially outer side in order to form, in the region of the upper side (10), a ring offset and spaced apart from a longitudinal axis (6) in order to enlarge an emission field for radiation.

11. The surgical robot (102) according to one of claims 1 to 10, **characterized in that** the cover fastening element (1), comprises polyoxymethylene as material, in particular consists entirely of this material, and preferably has a dark color, in order to absorb reflective light in the best possible manner and, in addition, increase the contrast to the navigation marker.

## Revendications

1. Robot chirurgical (102) pour une intervention chirurgicale sur un patient avec :
un bras de robot lié à une base de robot et une tête de robot (116) liée au bras de robot, dans lequel le bras de robot présente au moins un segment de bras de robot motorisé,
une pluralité de marqueurs de navigation qui sont agencés au niveau d'une surface de l'au moins un segment de bras de robot et/ou au niveau de la tête de robot (116), dans lequel ces marqueurs de navigation dépassent respectivement de la surface au moins partiellement, dans lequel au moins une quantité partielle des marqueurs de navigation comprend une interface de fixation de LED (114),
un film de recouvrement (2) stérile chirurgical qui est adapté de sorte qu'il puisse être agencé sur les marqueurs de navigation et
une pluralité d'éléments de fixation de recouvrement (1), dans lequel les éléments de fixation de recouvrement (1) coagissent avec le film de recouvrement (2) stérile chirurgical et sont conçus couplables et découplables de manière amovible au-dessus des marqueurs de navigation par leur interface de fixation de LED (114), dans lequel chacun des éléments de fixation de recouvrement (1) présente une ouverture (4) le long d'un axe longitudinal (6) depuis un côté inférieur (8) vers un côté supérieur (10) de l'élément de fixation de recouvrement (1) dans laquelle le marqueur de navigation peut être introduit,
**caractérisé en ce que**
chacun des éléments de fixation de recouvrement (1) présente une interface de fixation de recouvrement (12) configurée de manière complémentaire à l'interface de fixation de LED (114) dans la zone du côté inférieur (8) de l'élément de fixation de recouvrement (1) par le biais de laquelle l'élément de fixation de recouvrement (1) peut être fixé de manière amovible à force et/ou par complémentarité de formes et qui coagit avec l'interface de fixation de LED (114) afin de fixer et sécuriser une partie du film de recouvrement (2) stérile chirurgical aux marqueurs de navigation.

2. Robot chirurgical (102) selon la revendication 1, **caractérisé en ce que** l'élément de fixation de recouvrement (1) est configuré comme clip de fixation avec une liaison à enclenchement (14) comme interface de fixation de recouvrement (12), pour lequel au moins un, en particulier plusieurs, doigts d'encliquetage (16) s'étend en direction de l'axe longitudinal (6) vers le côté inférieur (8) et peut être dévié élastiquement en direction radiale afin de maintenir l'élément de fixation de recouvrement (1) à force et/ou à complémentarité de formes.

3. Robot chirurgical (102) selon la revendication 2, **caractérisé en ce que** le doigt d'encliquetage (16) présente une saillie d'encliquetage (20) terminale radialement vers l'intérieur pour une configuration d'une contre-dépouille pour une complémentarité de formes.

4. Robot chirurgical (102) selon la revendication 2 ou 3, **caractérisé en ce que** l'élément de fixation de recouvrement (1) présente des doigts d'encliquetage (16) en saillie vers son côté inférieur (8), agencés en particulier à symétrie de rotation, répartis uniformément autour de son ouverture qui garantissent une fixation circonférentielle homogène de l'élément de fixation de recouvrement (1), et les doigts d'encliquetage (16) configurent en particulier eux-mêmes le côté inférieur (8).

5. Robot chirurgical (102) selon l'une quelconque des revendications 1 à 4, **caractérisé en ce que** le marqueur de navigation présente un boîtier de LED (104) en forme de cylindre creux le long d'un axe longitudinal de LED et présente une rainure (112) circonférentielle complète comme interface de fixation de LED au niveau du côté extérieur radial du boîtier de LED (114) afin de configurer une contre-dépouille circonférentielle qui coagit avec les doigts d'encliquetage (16) élastiques radialement avec une saillie d'encliquetage (20) radialement vers l'intérieur de l'interface de fixation de recouvrement (12).

6. Robot chirurgical (102) selon l'une quelconque des revendications 1 à 5, **caractérisé en ce que** l'élément de fixation de recouvrement (1) est configuré comme clip de fixation avec une liaison d'encliquetage (14) comme interface de fixation de recouvrement (12), dans lequel une fente circonférentielle est configurée entre un boîtier de LED (114) du marqueur de navigation et l'élément de fixation de recouvrement (1), laquelle fente est conçue de sorte qu'un film de recouvrement (2) stérile soit reçu dans celle-ci avec un ajustement précis, en particulier la largeur de la fente correspond à l'épaisseur du film de recouvrement (2), de sorte que le film de recouvrement (2) soit serré entre l'élément de fixation de recouvrement (1) et le marqueur de navigation, en particulier une diode électroluminescente (101).

7. Robot chirurgical (102) selon l'une quelconque des revendications 1 à 6, **caractérisé en ce que** le film de recouvrement (2) est rigidement fixé au côté supérieur (10) de l'élément de fixation de recouvrement (1), de sorte que le film de recouvrement (2) stérile forme la barrière stérile vers l'extérieur.

8. Robot chirurgical (102) selon l'une quelconque des revendications 1 à 7, **caractérisé en ce que** l'élément de fixation de recouvrement (1) présente dans la zone de son côté supérieur (10) au moins une saillie, en particulier circonférentielle, radialement vers l'extérieur afin de configurer une contre-dépouille pour une manipulation manuelle.

9. Robot chirurgical (102) selon l'une quelconque des revendications 1 à 8, **caractérisé en ce que** l'élément de fixation de recouvrement (1) est conçu à symétrie de rotation et présente en direction de l'axe longitudinal (6) en particulier un formage conique,
dans lequel en particulier un diamètre extérieur au niveau du côté supérieur (10) est supérieur à un diamètre extérieur du côté inférieur (8) afin de fournir une bonne manipulation et/ou
dans lequel en particulier un diamètre intérieur au niveau du côté supérieur (10) est supérieur à un diamètre intérieur du côté inférieur (8) afin de garantir une bonne visibilité du marqueur de navigation.

10. Robot chirurgical (102) selon l'une quelconque des revendications 1 à 9, **caractérisé en ce que** l'élément de fixation de recouvrement (1) présente un niveau (22) radialement vers l'extérieur en direction de l'axe longitudinal (6) vers son côté supérieur (10) au niveau de son côté intérieur radial afin de configurer dans la zone du côté supérieur (10) un anneau espacé en déport par rapport à un axe longitudinal (6) afin d'agrandir un champ d'émission pour un rayonnement.

11. Robot chirurgical (102) selon l'une quelconque des revendications 1 à 10, **caractérisé en ce que** l'élément de fixation de recouvrement (1) présente comme matériau des polyoxyméthylènes, en particulier se compose dans l'ensemble de celui-ci et de préférence présente une couleur sombre afin d'absorber au mieux de la lumière réfléchissante et d'augmenter en outre un contraste par rapport au marqueur de navigation.
